Europäisches Patentamt

European Patent Office

Office européen des brevets

(11) Veröffentlichungsnummer: **0 143 451**
**A2**

(12) **EUROPÄISCHE PATENTANMELDUNG**

(21) Anmeldenummer: **84114214.4**

(22) Anmeldetag: **24.11.84**

(51) Int. Cl.⁴: **B 44 B 5/00**

(30) Priorität: **29.11.83 CH 6376/83**

(43) Veröffentlichungstag der Anmeldung: **05.06.85**
**Patentblatt 85/23**

(84) Benannte Vertragsstaaten: **AT BE FR GB IT LU NL SE**

(71) Anmelder: **Tecnomara AG, Rieterstrasse 59,**
**CH-8002 Zürich (CH)**

(72) Erfinder: **Fischer, Thomas, Rieterstrasse 59,**
**CH-8002 Zürich (CH)**

(74) Vertreter: **Schmauder, Klaus Dieter et al, c/o Schmauder**
**& Wann Patentanwaltsbüro Nidelbadstrasse 75,**
**CH-8038 Zürich (CH)**

(54) **Gerät zum fortlaufenden Beschriften von Petrischalen.**

(57) Das Gerät weist eine blockartig ausgebildete Beschriftungsvorrichtung (8) auf, welche am Umfang der Schale (4) einer Petrischale (2) angreift. Ein weiches Förder- und Andrückband (44) drückt die Schale (4) gegen die Beschriftungsvorrichtung (8) und rollt diese an der Beschriftungsvorrichtung (8) ab. Die Beschriftungsvorrichtung weist vorzugsweise für jedes Schriftzeichen (40) einen auswechselbaren Prägestempel (36) auf, der auf einem Heizstab (40) aufliegt. Damit ergibt sich eine einfache sichere Beschriftung des Umfanges der Schale (4) einer Petrischale (2), die mit einem Deckel (6) versehen ist.

- 1 -

**Gerät zum fortlaufenden Beschriften von Petrischalen**

Die Erfindung betrifft ein Gerät zum fortlaufenden Beschriften von Petrischalen gemäss Oberbegriff des Anspruches 1.

Es sind Geräte zum fortlaufenden Beschriften von Petrischalen bekannt, bei dem solche Schalen am Boden oder am Deckel dadurch beschriftet werden, dass sie mit aufgedruckten Zeichen oder mit einem Etikett versehen werden. Diese Art der Beschriftung erbringt jedoch entscheidende Nachteile. Zum einen sind am Boden angebrachte Beschriftungen schwer zu lesen und werden durch Verschmutzung häufig unleserlich. Eine Beschriftung des Deckels birgt zum anderen die Gefahr in sich, dass solche Deckel unter Umständen nicht auf die zugehörige Schale aufgebracht werden, sodass Verwechslungen entstehen. Wesentlicher ist es jedoch noch, dass beim Aufbringen der Beschriftungen die Gefahr besteht, dass die Schale und/oder der Deckel der Petrischale zerstört werden/wird. Dies bedingt nicht nur Ausschuss, sondern bei gefüllten Petrischalen entsteht eine Verschmutzung des Gerätes mit der Nährlösung.

Aufgabe der Erfindung ist es, ein Gerät der eingangs genannten Art zu schaffen, mit dem die erwähnten Nachteile vermieden werden.

Diese Aufgabe wird erfindungsgemäss durch die kennzeichnenden Merkmale des Anspruches 1 gelöst.

Dadurch, dass das Gerät die Beschriftung am Umfang der Schale anbringt, wird insbesondere eine Beschädigung oder Zerstörung der Schale und/oder des Deckels vermieden, da der Umfang der Schale wesentlich weniger druckempfindlich ist als die Boden- oder die Deckelfläche. Weiter kommt hinzu, dass die Beschriftung in jedem Falle besser erkennbar ist, als eine Beschriftung des Bodens oder aber auch des Deckels, und insbesondere ist eine einwandfreie Zuordnung der Beschriftung einer gefüllten Petrischale auch dann gegeben, wenn die Deckel von Petrischalen verwechselt werden. Schliesslich ist das Gerät auch äusserst einfach, da die Beschriftungsvorrichtung ohne Farbwerk arbeitet und die heizbaren Schriftzeichen das Schriftbild direkt in die Schale aus Kunststoff einschmelzen.

Vorteilhafte Ausgestaltungen des Gerätes sind in den Ansprüchen 2 bis 8 beschrieben.

Eine besonders schonende Förderung und Anpressung ergibt sich bei einer Ausgestaltung des Gerätes nach Anspruch 2.

Die Schriftzeichen können parallel zur Achse der Petrischale am Umfang derselben angreifen. Vorteilhaft ist jedoch eine Ausgestaltung nach Anspruch 3, da dann die Schriftzeichen mithelfen, die Schale auf der Auflage zu halten.

Eine sehr einfache Ausgestaltung des Gerätes ergibt sich, wenn die Schriftzeichen gemäss Anspruch 4 an einem auswechselbaren Stempel angeordnet sind. Durch einfaches Auswechseln des Stempels können dann die Schriftzeichen geändert werden. Besonders vorteilhaft ist eine Ausgestaltung des Gerätes nach Anspruch 5, da hier keine Stempel auszuwechseln sind, sondern lediglich das Schriftzeichenband entsprechend umgestellt werden muss. Diese Lösung gestattet insbesondere auch einen ferngesteuerten Betrieb und erleichtert das Beschriften mit einer fortschreitenden Seriennummer.

0143451

Eine Ausbildung des Gerätes nach Anspruch 6 gestattet eine besonders einfache Anbringung und Auswechslung der Präge- stempel.

Damit insbesondere auch leichte Petrischalen, d.h. solche mit geringer oder fehlender Füllung einwandfrei beschriftet wer- den können, ist eine Ausgestaltung des Gerätes nach Anspruch 7 von Vorteil.

In der Regel wird ein solches Beschriftungsgerät in eine Produktionsstrasse eingegliedert. Dabei ist es von Vorteil, wenn das Gerät nach Anspruch 8 ausgestaltet ist. Sollten bei ei- nem solchen Gerät die Schriftzeichen aus irgendeinem Grunde fehlen, so gewährleistet das Gerät dennoch einen fortlaufen- den Durchlauf der Petrischalen, wenngleich sie denn auch nicht beschriftet sind. Auf diese Weise wird ein Stau im Her- stellungs- bzw. Füllprozess von Petrischalen vermieden.

Ein solches Gerät kann beliebig eingesetzt werden. Besonders vorteilhaft ist seine Anordnung unmittelbar im Anschluss an eine Füllvorrichtung für Petrischalen, da dann eine einwand- freie Zuordnung der Beschriftung zu den gefüllten Petrischa- len möglich ist und Verwechslungen vermieden werden. Die ge- füllten und beschrifteten Petrischalen können dann in übli- cher Weise weitertransportiert und gelagert werden.

Ausführungsbeispiele des Gegenstandes der Erfindung werden nachfolgend anhand von Zeichnungen näher beschrieben, dabei zeigen:

| | |
|---|---|
| Figur 1 | ein Gerät mit einem Beschriftungsblock aus einzelnen Prägestempeln im Grundriss; |
| Figur 2 | das Gerät der Figur 1 im Schnitt II-II der Figur 1; |
| Figur 3 | einen Beschriftungsblock mit an Bändern angeordneten Beschriftungszeichen für eine Vorrichtung der Figur 1, im Grundriss; und |
| Figur 4 | den Beschriftungsblock der Figur 3 im Schnitt IV-IV der Figur 3. |

Das in den Figuren beschriebene Gerät dient zum Beschriften von Petrischalen 2, die eine Schale 4 aufweisen, welche mit einem Deckel 6 versehen ist. Solche Petrischalen werden vorzugsweise aus glasklarem Kunststoff hergestellt.

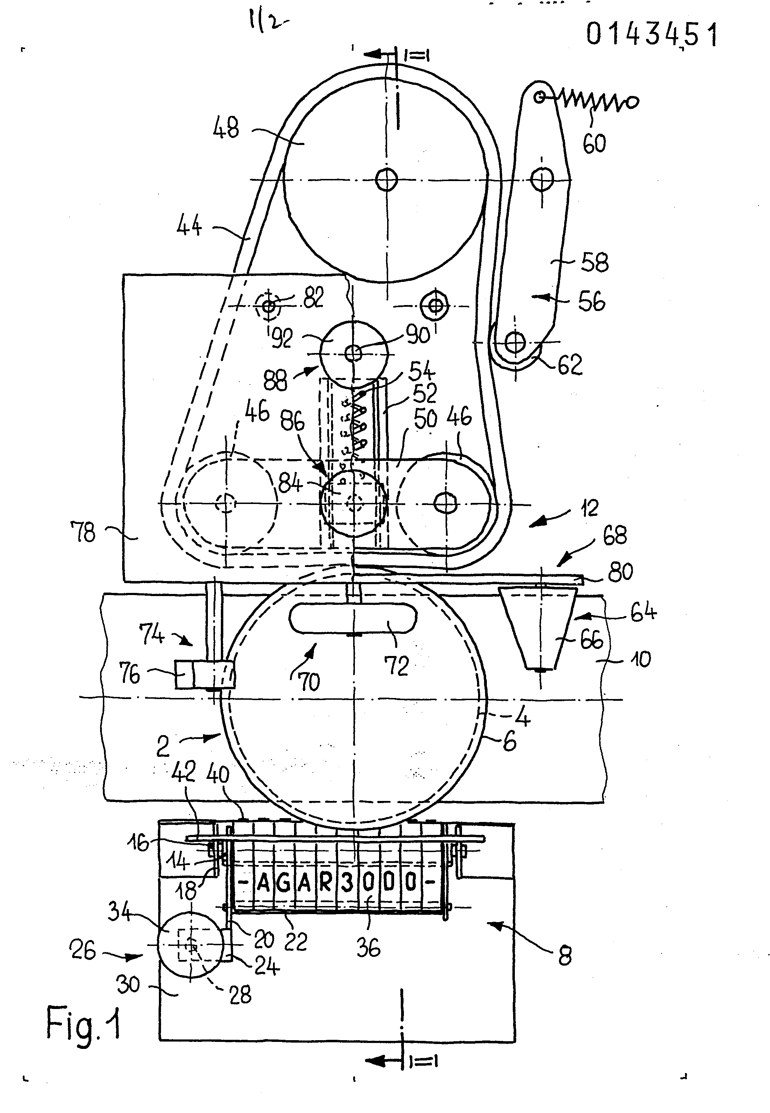

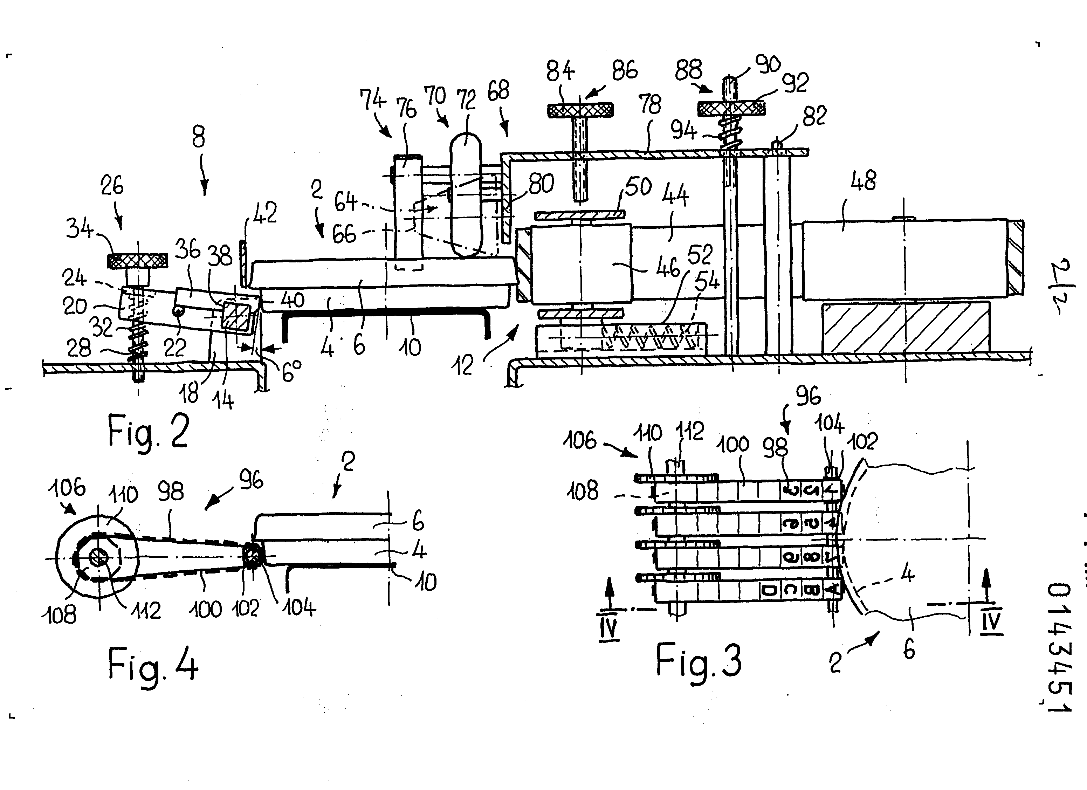

Das Gerät der Figuren 1 und 2 weist eine Beschriftungsvorrichtung 8 auf, die in Form eines feststehenden Blockes ausgebildet ist und längs der die Petrischalen fortlaufend über eine Auflage 10 und eine Fördervorrichtung 12 entlangbewegt werden. Die Fördervorrichtung 12 dient gleichzeitig als Andrückvorrichtung zum Andrücken der Petrischale 2 gegen die Beschriftungsvorrichtung 8. Die Petrischale 2 rollt dann an der Beschriftungsvorrichtung 8 ab.

Die Beschriftungsvorrichtung 8 enthält einen im Querschnitt rechteckigen Heizstab 14, der mittels Lagerzapfen 16 in Haltearmen 18 verschwenkbar gelagert ist. Am Heizstab 14 sind Arme 20 befestigt, die an ihrem dem Heizstab 14 abgewandten Teil mittels einer Querstange 22 verbunden sind. Ein Arm 20 ist verlängert und weist einen Flanschteil 24 auf, der zu einer Stellvorrichtung 26 gehört, mit der die Winkellage des Heizstabes 14 und damit auch der Arme 20 eingestellt werden kann. Die Stellvorrichtung enthält eine Stellschraube 28, die mit einem Gestell 30 verschraubt ist. Eine zwischen Gestell 30 und Flanschteil 24 eingespannte Feder 32 presst den Flanschteil 24 gegen eine Stellscheibe 34 der Stellschraube 28 vor. Am Heizstab 14 und an der Querstange 22 sind Prägestempel 36 aufgelegt. Letztere weisen eine Ausnehmung 38 auf, mit der sie den Heizstab 14 übergreifen, sodass sich die Prägestempel 36 in ihrer Längsrichtung an dem Heizstab 14 abstützen. Die Prägestempel 36 sind an ihrer vorderen Stirnseite mit zum Prägen geeigneten Schriftzeichen 40 versehen. Diese Schriftzeichen sind auf der Oberseite der Prägestempel 36 nochmals wiederholt, um jeweils feststellen zu können, welche Beschriftung eingestellt ist. Die Prägestempel 36 sind mit solcher Neigung angeordnet, dass ihre Schriftzeichen 40 gegen die Auflage 10 weisen, auf der die Petrischale 2 angeordnet ist. Der Neigungswinkel beträgt vorzugsweise $6^{o}$. Dadurch wird erreicht, dass die an den Prägestempeln 36 abrollende Petrischale gegen die Auflage 10 gepresst wird.

Eine oberhalb der Prägestempel 36 angeordnete Stützvorrichtung 42 dient zum Abstützen des Deckels 6 der Petrischale 2, wenn in der Beschriftungsvorrichtung 8 keine Prägestempel 36 vorhanden sind. Die Petrischale kann dann das Gerät durchlaufen ohne beschriftet zu werden.

Die Fördervorrichtung 12 weist ein umlaufendes Förderband 44 auf, welches auf der der Beschriftungsvorrichtung 8 gegenüberliegenden Seite der Auflage 10 angeordnet und längs der Beschriftungsvorrichtung 8 verläuft. Das Förderband 44 ist um zwei Umlenkrollen 46 geführt und wird von einer Antriebsrolle 48 angetrieben. Die Umlenkrollen 46 sind in einem Halter 50 gelagert, der über eine Führung 52, die als Prismenführung ausgebildet ist, quer zur Auflage 10 verschiebbar ist. Eine Feder 54 spannt den Halter 50 in Richtung zur Auflage 10 bzw. Beschriftungsvorrichtung 8 vor. Am Förderband 44 greift weiter eine Ausgleichsvorrichtung 56 an, um das Förderband 44 gespannt zu halten. Die Ausgleichsvorrichtung 56 enthält eine Wippe 58, die mittels einer Feder 60 vorgespannt ist und eine Rolle 62 gegen das Förderband 44 drückt. Das Förderband besteht vorzugsweise aus Schaumstoff, damit es sich der Umfangsform der Petrischale 2 aus der Schale 4 und dem Deckel 6 anpassen kann. Das Förderband 44 presst die Petrischale 2 über den Deckel 6 gegen die Beschriftungsvorrichtung 8 vor.

Das Gerät ist überdies noch mit einer Vorrichtung 64 zum Schliessen allfällig nicht ganz korrekt auf der Schale 4 aufsitzender Deckel 6 versehen. Hierzu dient eine kegelstumpfförmige Rolle 66, die an einem heb- und senkbaren Halter 68 angeordnet und in Durchlaufrichtung gesehen hinter der Beschriftungsvorrichtung 8 positioniert ist.

- 7 -

An dem gleichen Halter 68 ist ferner ein Niederhalter 70 in Form einer Andrückrolle 72 vorhanden, welche auf den Deckel 6 der Petrischale 2 drückt. Ein weiterer, nachgeschalteter Niederhalter 74, in Form eines Schwenkhebels 76 ist ebenfalls am Halter 68 angeordnet und verhindert ein Abheben des Deckels, wenn die Petrischale 2 die Andrückrolle 72 verlässt. Der Halter 68 weist eine abgewinkelte Platte 78 auf, die an ihrem der Auflage 10 zugewandten Ende einen Winkelteil 80 enthält, an dem die Rolle 66, die Andrückrolle 72 und der Schwenkhebel 76 befestigt sind. Die Platte 78 ist am rückwärtigen Ende an Zapfen 82 verschwenkbar gelagert. Eine Rändelschraube 84 dient als Höhenstellvorrichtung für die am Winkelteil 80 angeordneten Aggregate. Eine Vorspannvorrichtung 88 für diese Aggregate weist einen am Gestell 30 befestigten Gewindebolzen 90 auf, der die Platte 78 durchdringt und eine Rändelmutter 92 trägt, welche eine Feder 94 gegen die Platte 78 vorspannt.

Die mittels des Heizstabes 14 aufgeheizten Prägestempel 36 schmelzen den Werkstoff der Schale 4 an und liefern so eine Prägebeschriftung, die auf einfache Weise herstellbar und unverwischbar ist. Die Prägestempel 36 können auf einfachste Art und Weise gegen Prägestempel anderer Schriftzeichen ausgewechselt werden. Ein nicht näher dargestellter Thermostat regelt die Wärmezufuhr zu den Prägestempeln.

Die Figuren 3 und 4 zeigen eine abgewandelte Beschriftungsvorrichtung 96, welche für das Gerät der Figuren 1 und 2 geeignet ist. Die Schriftzeichen 98 sind dabei jeweils an
Schriftzeichenbändern 100 angeordnet, die quer zur Beschriftungsebene verlaufen. Am vorderen, der Auflage 10 zugewandten
Ende sind die Schriftzeichenbänder 100 über Schwenkglieder
102 geführt, die an einem Heizstab 104 drehbar angeordnet
sind. Auf der der Auflage 10 abgewandten Seite sind die
Schriftzeichenbänder 100 über Stellvorrichtungen 106 geführt,
welche eine prismatische Umlenkrolle 108 aufweisen, die jeweils mit einem von Hand zu betätigenden Stellrad 110 verbunden sind. Die Umlenkrolle 108 und das Stellrad 110 sind
auf einer Achse 112 drehbar gelagert. Anstelle des von Hand
zu betätigenden Stellrades 110 können auch Stellräder vorgesehen sein, die motorisch getrieben und mit einer Steuervorrichtung verbunden sein können, um eine automatische Einstellung der Beschriftungsvorrichtung 96 zu ermöglichen.

**Bezugszeichenliste**

| | |
|---|---|
| 2 | Petrischale |
| 4 | Schale |
| 6 | Deckel |
| 8 | Beschriftungsvorrichtung |
| 10 | Auflage |
| 12 | Fördervorrichtung |
| 14 | Heizstab |
| 16 | Lagerzapfen |
| 18 | Haltearm |
| 20 | Arm |
| 22 | Querstange |
| 24 | Flanschteil |
| 26 | Stellvorrichtung |
| 28 | Stellschraube |
| 30 | Gestell |
| 32 | Feder |
| 34 | Stellscheibe von 28 |
| 36 | Prägestempel |
| 38 | Ausnehmung |
| 40 | Schriftzeichen |
| 42 | Stützvorrichtung |
| 44 | Förderband |
| 46 | Umlenkrolle |
| 48 | Antriebsrolle |
| 50 | Halter |
| 52 | Führung |
| 54 | Feder |

0143451

| | |
|---|---|
| 56 | Ausgleichsvorrichtung |
| 58 | Wippe |
| 60 | Feder |
| 62 | Rolle |
| 64 | Vorrichtung zum Deckelschliessen |
| 66 | kegelstumpfförmige Rolle |
| 68 | Halter |
| 70 | Niederhalter |
| 72 | Andrückrolle |
| 74 | Niederhalter |
| 76 | Schwenkhebel |
| 78 | abgewinkelte Platte |
| 80 | Winkelteil |
| 82 | Zapfen |
| 84 | Rändelschraube |
| 86 | Höhenstellvorrichtung |
| 88 | Vorspannvorrichtung |
| 90 | Gewindebolzen |
| 92 | Rändelmutter |
| 94 | Feder |
| 96 | Beschriftungsvorrichtung |
| 98 | Schriftzeichen |
| 100 | Schriftzeichenband |
| 102 | Schwenkglied |
| 104 | Heizstab |
| 106 | Stellvorrichtung |
| 108 | Umlenkrolle |
| 110 | Stellrad |
| 112 | Achse |

0143451

Patentansprüche
_____

1. Gerät zum fortlaufenden Beschriften von Petrischalen aus
   Kunststoff, bei denen Schalen mit einem losen Deckel versehen sind, mit einer Auflage für die Petrischalen und einer Beschriftungsvorrichtung, dadurch gekennzeichnet, dass
   der Auflage (10) für die Petrischalen (2) auf einer Seite
   die Beschriftungsvorrichtung (8, 96) mit in Form eines
   ortsfesten Blockes angeordneten heizbaren Schriftzeichen
   (40, 98) zugeordnet ist, und auf der anderen Seite ein
   parallel zur Auflage (10) verlaufendes weiches Förder- und
   Andrückband (44) zugeordnet ist, welches am Deckel (6)
   angreift und die Petrischale (2) mit ihrer Schale (4) an
   den Schriftzeichen (40, 98) abrollend vorbeibewegt, wobei
   mindestens im Bereich der Beschriftungsvorrichtung (8, 96)
   ein die Petrischale (2) gegen die Auflage (10) drückender
   Niederhalter (70) vorhanden ist.

2. Gerät nach Anspruch 1, dadurch gekennzeichnet, dass das
   Förder- und Andrückband (44) als Schaumstoffband ausgebildet ist.

3. Gerät nach Anspruch 1, dadurch gekennzeichnet, dass die
   Schriftzeichen (40) gegen die Auflage (10) geneigt sind,
   wobei die Neigung vorzugsweise mittels einer Stellvorrichtung (26) einstellbar ist.

4. Gerät nach Anspruch 1, dadurch gekennzeichnet, dass die
   Schriftzeichen (40) an einem auswechselbaren Prägestempel (36) angeordnet sind.

5. Gerät nach Anspruch 1, dadurch gekennzeichnet, dass die Schriftzeichen (98) jeweils an einem quer zur Beschriftungsebene verlaufenden Schriftzeichenband (100) mit verschiedenen Schriftzeichen angeordnet sind, wobei jedem Schriftzeichenband (100) eine Stellvorrichtung (106) zum Umstellen der Schriftzeichen zugeordnet ist.

6. Gerät nach Anspruch 4, dadurch gekennzeichnet, dass jeder Prägestempel (36, 116) eine Ausnehmung (38) aufweist, mit der er auf einem Heizstab (14) aufliegt, wobei der Prägestempel (36) vorzugsweise gegen die Auflage (10) geneigt ist.

7. Gerät nach Anspruch 1, dadurch gekennzeichnet, dass der Niederhalter (70) mittels einer Stellvorrichtung (86, 88) in seiner Höhenlage und/oder Vorspannung einstellbar ist.

8. Gerät nach Anspruch 1, dadurch gekennzeichnet, dass es eine der Beschriftungsvorrichtung (8) zugeordnete Stützvorrichtung (42) zum Abstützen vorzugsweise des Deckels (6) der Petrischale (2) bei fehlenden Schriftzeichen (40) aufweist.

Fig. 1

2/2

0143451

Fig. 2

Fig. 3

Fig. 4